# EUROPÄISCHE PATENTSCHRIFT

(11) **EP 1 708 766 B1**
(45) Veröffentlichungstag und Bekanntmachung des Hinweises auf die Patenterteilung: **29.07.2015**
(21) Anmeldenummer: 05700319.6
(22) Anmeldetag: 18.01.2005
(51) Int. Cl.: A61M 1/06

(54) **BRUSTHAUBENEINSATZ UND BRUSTHAUBE ZUR VERWENDUNG DES BRUSTHAUBENEINSATZES**
BREAST CAP PART AND BREAST CAP FOR USING THE BREAST CAP PART
EMBOUT POUR TETERELLE ET TETERELLE PERMETTANT D'UTILISER UN TEL EMBOUT POUR TETERELLE

(30) Priorität: 27.01.2004 CH 109042004
(43) Veröffentlichungstag der Anmeldung: 11.10.2006
(73) Patentinhaber: Medela Holding AG, 6340 Baar (CH)
(72) Erfinder: RÖLLIN, Richard, CH-6313 Menzingen (CH)
(74) Vertreter: Clerc, Natalia
(86) Internationale Anmeldenummer: PCT/CH2005/000024
(87) Internationale Veröffentlichungsnummer: WO 2005/070476

(56) Entgegenhaltungen:
- WO-A-02/17993
- US-A- 5 897 580
- US-A1- 2002 198 489
- US-A1- 2003 073 951
- US-A1- 2004 087 898
- US-B1- 6 358 226

## Beschreibung

### Technisches Gebiet

Die Erfindung betrifft einen Brusthaubeneinsatz für eine Brustpumpe gemäss Oberbegriff des Patentanspruchs 1 sowie 2 und ein Brusthaubenset einer Brustpumpe gemäss Oberbegriff des Patentanspruchs 8 sowie 9.

### Stand der Technik

Brustpumpen sind bekannt. Sie weisen üblicherweise eine Vakuumpumpe, eine damit verbundende Brusthaube zur Auflage auf eine Areola einer menschlichen Mutterbrust sowie einen mit der Brusthaube verbundenen Sammelbehälter zum Sammeln der abgepumpen Milch auf.

Die bekannten Brusthaube bestehen im wesentlichen aus einem starren Trichter, so dass Brusthaubeneinsätze verwendet werden, um eine Anpassung an die unterschiedlichen Brustgrössen zu ermöglichen.

Diese bekannten Brusthaubeneinsätze weisen die unterschiedlichsten Formen auf. US-A-5'941'847 offenbart beispielsweise einen trichterförmigen Einsatz mit einem langgezogenen Trichterhals. EP-A-0'237'474 zeigt einen Einsatz mit einem zylinderförmigen Nippelansatz. Dieser Einsatz ist aus einem Material gefertigt, welches bezüglich Wasser bis zu einer Temperatur von 100°C chemisch inaktiv ist.

US-A-5'100'406 und US-A-5'885'246' beschreiben kegelstumpfartige Einsätze mit verdickten bzw. verdünnten Zonen, welche eine verbesserte Stimulierung der Mutterbrust gewährleisten sollen, um so den Abpumpprozess zu optimieren.

WO 02/17993 zeigt einen Brusthaubeneinsatz, welcher einen angenehmen Tragkomfort gewährleistet. Der Einsatz weist einen kegelstumpfartigen Grundkörper mit einer innerren und einer äusseren Kegelwand auf, welche die Wände einer geschlossenen Kammer bilden. Die Kammer ist mit Luft, einem Gas, einem Gel oder Silikon gefüllt und die innere Kegelwand ist elastisch deformierbar ausgebildet. Dadurch passt sich der Brusthaubeneinsatz optimal an die Form der Mutterbrust an, ohne einen unerwünschten Druck auf diese auszuüben.

Des weiteren offenbart US-A-5'776'177 eine c-förmige Wärme- oder Kühlpackung zur therapeutischen Verwendung an vorstehenden Körperteilen wie beispielsweise an einem Ellbogen, an einer Schulter oder an einer Brust. US'177 offenbart des weiteren eine Brustpumpe, bei welcher der Haubentrichter mit einem Kragen versehen ist, welcher aus dieser Wärme- oder Kühlpackung besteht. Der Trichter umgibt somit bei Gebrauch die Areola der Mutterbrust und der durch die c-förmige Wärme- oder Kühlpackung gebildete Flansch liegt ausserhalb der Areola an der Brust an, damit diese gewärmt bzw. gekühlt wird. Dadurch sollen sich Schwellungen und Reizungen der Brust verhindern lassen.

US 2002/0198489 offenbart diverse Beispiele von Brusthauben mit einem Hohlraum, welcher mit warmem Wasser oder mit einem warmen Gel gefüllt werden kann.

US 5 897 580 zeigt einen Pad mit einer Substanz, welche eine exotherme Reaktion durchführen kann. Dieses Pad ist c-formig ausgebildet und kann entweder aussen auf der Brusthaube angeordnet sein oder alternativ auf ihrer Innenseite anliegen.

US 2003/0073951 beschreibt eine komplex aufgebaute Brusthaube mit einem beheizbaren Teil in Form von blütenförmigen Blättern Dieser Teil wird von einer Hülle überdeckt.

### Darstellung der Erfindung

Es ist eine Aufgabe der Erfindung, einen Brusthaubeneinsatz für eine Brustpumpe zu schaffen, welcher einerseits den Tragkomfort weiter verbessert und zudem einen positiven Einfluss auf den Abpumpprozess ausübt.

biese Aufgabe löst ein Brusthaubeneinsatz mit den Merkmalen des Patentanspruchs 1 bzw. 2.

Es ist eine weitere Aufgabe der Erfindung, eine Brusthaube einer Brustpumpe zu schaffen, welche zur Verwendung mit derartig verbesserten Brusthaubeneinsätzen geeignet ist.

Diese Aufgabe löst ein Brusthaubenset mit den Merkmalen des Patentanspruchs 8 bzw. 9.

Der erfindungsgemässe Brusthaubeneinsatz, welcher im Gebrauch zum Anliegen an eine Areola einer weiblichen Brust vorgesehen ist, weist einen kegelstumpfförmigen Grundkörper mit einer grossen hinteren Öffnung und einer kleinen vorderen Öffnung auf, wobei der Grundkörper zur Aufnahme in einen Trichter einer Brusthaube ausgebildet ist Erfindungsgemäss ist der kegelstumpfförmige Grundkörper mindestens teilweise aus einem wärmespeichernden und/oder wärmeleitenden Material gefertigt und er ist im Gebrauch erwärmt. Dabei erfolgt diese Erwärmung nicht ausschliesslich durch Übermittlung von Körpertemperatur der weiblichen Brust, sondern es werden andere Mittel zur Erwärmung des Brusthaubeneinsatzes eingesetzt.

In einer Ausführungsform ist der Einsatz ähnlich oder gleich wie in WO 02/17993 beschrieben ausgebildet, wobei seine Klammer mit einem Füllmaterial versehen ist, welches gute wärmespeichernde Eigenschaften aufweist. Dieser Einsatz lässt sich vor seiner Verwendung in einem Wasserbad, in einem Ofen, durch Heissluft oder durch Auflage auf einer Heizfläche auf eine gewünschte Temperatur erhitzen. Bei Gebrauch, d.h. während dem Abpumpen, gibt er serine Wärme an die Mutterbrust ab.

Gemäss der Erfindung lässt sich der Einsatz während seines Gebrauchs bzw. dank seiner Auflage in der Brusthaube mittels dieser erwärmen bzw. der Einsatz ist selber mit Heizelementen versehen.

Da der erfindungsgemässe Brusthaubeneinsatz bei Gebrauch auf eine gewünschte Temperatur erwärmt ist, erhöht sich der Tragkomfort. Die Mutter muss nicht einen kalten Einsatz an die empfindliche Brust legen, sondern kann eine ihr angenehm erscheinende Temperatur wählen.

Des weiteren wird der natürliche Stillvorgang stärker berücksichtigt, da ja auch-ein an die Mutterbrust gehaltener Säugling insbesondere im unmittelbaren Bereich der Brustwarze Wärme abgibt. Durch diese wärmetechnische Simulation des Stillvorgangs wird die Mutterbrust optimal stimuliert und so der Abpumpvorgang optimiert.

Ein erhöhter Tragkomfort und eine Verbesserung des Abpumpvorgang kann auch mit einem Brusthaubeneinsatz erzielt werden, dessen kegelförmiger Grundkörper aus einem Schaumstoff oder aus einem schaumstoffähnlichen Material, insbesondere aus Polyurethan-Schaum oder aus einem Polyolefine-Schaum, gefertigt ist. Dieses Material ist relativ weich und deshalb angenehm zu tragen. Zudem weist es einen relativ geringen Wärmeleitkoeffizienten auf, so dass es sich auf der Haut warm anfühlt.

Weitere vorteilhafte Ausführungsformen gehen aus den abhängigen Patentansprüchen hervor.

### Kurze Beschreibung der Zeichnungen

Im folgenden wird der Erfindungsgegenstand anhand von bevorzugten Ausführungsbeispielen, welche in den beiliegenden Zeichnungen dargestellt sind, erläutert. Es zeigen:
- Figur 1: einen Vertikalschnitt durch eine erste Ausführungsform eines Brusthaubeneinsatzes mit einem doppelwandigen Grundkörper;
- Figur 2: einen Vertikalschnitt durch eine Brusthaube und einen Brusthaubeneinsatz mit einem Widerstandsheizelement gemäss einer ersten Ausführungsform der Erfindung;
- Figur 3: einen Vertikalschnitt durch eine Brusthaube und einen Brusthaubeneinsatz mit einem Auslösemittel gemäss einer zweiten Ausführungsform der Erfindung und
- Figur 4: einen Vertikalschnitt durch eine vierte Ausführungsform eines erfindungsgemässen Brusthaubeneinsatzes.

### Wege zur Ausführung der Erfindung

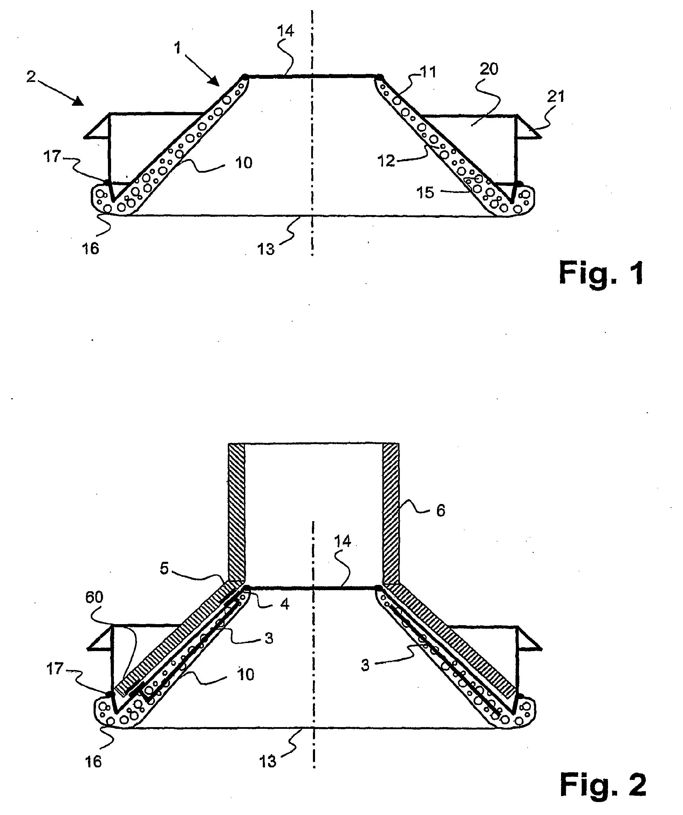

In Figur 1 ist eine Ausführungsform eines Brusthaubeneinsatzes dargestellt.

Er weist einen kegelstumpfförmigen Grundkörper 1 mit einer inneren und -einer äusseren Kegelwand 10, 11 auf. Die Kegelwände 10, 11 sind vorzugsweise aus einer Kunststofffolie oder einem anderen geeigneten gummielastischen Material gefertigt. Die Folie der äüsseren Kegelwand 11 weist vorzugsweise eine Dicke von circa 0.4 mm und die Folie der inneren Kegelwand 10 eine Dicke von circa 0.1 bis 25 mm auf.

Mindestens die innere Kegelwand 10 ist elastisch verformbar. Vorzugsweise sind beide Kegelwände 11 elastisch verformbar. Die zwei Kegelwände 10, 11 sind so beabstandet miteinander verbunden, dass sie eine geschlossene Kammer 12 umschliessen. Vorzugsweise sind die zwei Kegelwände 10, 11 miteinander verschweisst, wobei sie bei Gebrauch eine der stillenden Mutter zugewandte hintere grosse Öffnung 13 und eine vordere kleine Öffnung 14 freilassen. Die Öffnungen 13, 14 sind vorzugsweise rund. Die kleine Öffnung 14 dient dazu, die Brustwarze der Mutterbrust freizulassen.

Damit die Schweissnähte die Brust nicht verletzen oder irritieren, ist insbesondere im Bereich der grossen Öffnung die Folie der inneren Kegelwand 10 über die Folie der äusseren Kegelwand 11 gestülpt, so dass ein weiches Auflagepolster 16 gebildet ist und sich die Schweissnaht 17 auf der Aussenseite des Einsatzes befindet. Das Polster 16 steht bei Gebrauch dadurch einem Trichter einer Brusthaube vor.

Zur Befestigung des Einsatzes an einer Brusthaube einer Brustpumpe ist am Grundkörper 1 im Bereich seiner grossen Öffnung 13 ein umlaufender Kragen 2 angeformt. Dieser Kragen 2 besteht vorzugsweise aus einer deformierbaren Folie. Sie kann an der Schweissnaht der zwei Kegelwände 10, 11 angeschweisst sein. Es kann jedoch auch eine der zwei Kegelwände 10, 11 entsprechend länger ausgebildet sein. Vorzugsweise ist der Kragen 2 V-förmig ausgebildet. Ein erster Schenkel 20 des Kragens 2 liegt am Trichter der Brusthaube an, ein zweiter Schenkel 21 steht davon ab und ist zwecks Entfernung des Einsatzes von der Haube gut von Hand ergreifbar.

In der Kammer 12 ist ein deformierbares Füllmedium 15 vorhanden. Vorzugsweise ist die Kammer 12 vollständig mit diesem Medium 15 ausgefüllt. Erfindungsgemäss ist das Medium 15 ein wärmespeicherndes und/oder wärmeleitendes Material. Vorzugweise ist es ein Gel, insbesondere ein medizinisches Paraffin- oder Weissöl, oder eine verdickte Flüssigkeit, welches bzw. welche derartige Eigenschaften aufweist.

Erfindungsgemäss ist nun der Einsatz bzw. zumindest sein Grundkörper 1 bei bzw. vor seinem Gebrauch erwärmt. Diese Erwärmung kann auf unterschiedliche Weise erfolgen.

Bei Erwärmung vor Gebrauch wird der Einsatz beispielsweise in einem Wasserbad auf die gewünschte Temperatur erwärmt. Der Einsatz kann auch in einen Ofen gelegt oder mit Heissluft beaufschlagt werden. Des weiteren kann der Einsatz auf ein Wärmeelement gelegt und durch Wänneleitung aufgewärmt werden. Dieses Wärmeelement ist beispielsweise Teil der Brustpumpe und vorzugsweise bereits entsprechend der Form des Einsatzes ausgebildet. Beispielsweise ist das Wärmeelement als kegelstumpfförmige Auflage ausgebildet, so dass der Einsatz zwecks Erwärmung auf das Wärmeelement gestülpt werden kann. Um den Wärmprozess zu beschleunigen, ist vorzugsweise zusätzlich ein Deckel mit einer kegelstumpfförmigen Innenform vorhanden, welcher auf den aufgestülpten Einsatz gelegt wird, so dass Deckel und Heizelement einen geschlossenen Hohlraum zur Aufnahme des Einsatzes bilden. Der Deckel selber ist vorzugsweise zusätzlich oder anstelle der kegelstumpfförmigen Auflage mit Heizelementen versehen.

Wird der Einsatz auf die oben beschriebene Weise erwärmt, ist er, bzw. sein Füllmedium, mindestens teilweise aus einem Material gefertigt, welches gute wärmespeichernde Eigenschaften aufweist.

In der in Figur 2 dargestellten ersten Ausführungsform der Erfindung kann der Einsatz vor oder während seinem Gebrauch erwärmt werden. Hierfür ist im Grundkörper 1 mindestens ein Widerstandsheizelement, hier in Form eines Widerstandsheizdrahtes 3, angeordnet. Dieser Widerstandsheizdraht 3 ist mit elektrischen Kontaktmitteln 4, vorzugsweise Kontaktflächen, verbunden, welche auf der Aussenseite des Einsatzes angebracht sind. Diese Kontaktmittel 4 kontaktieren elektrische Kontaktelemente 5, welche an der Innenfläche der Brusthaube 6 angebracht sind und welche über elektrische Leitungen mit der Brustpumpe verbunden sind. Wird der Einsatz in die Brusthaube 6 eingelegt, wird der Kontakt geschlossen und die Widerstandsheizelemente werden über die Stromversorgung der Brustpumpe erhitzt, so dass sich der Grundkörper 1 des Einsatzes erwärmt. Wird der Einsatz während dem Gebrauch der Brustpumpe erwärmt, ist eine gute Speicherkapazität nicht zwingend notwendig. In diesem Fall ist es wichtiger, dass eine gute Wärmeleitung vom erwärmten Grundkörper zur Mutterbrust vorhanden ist.

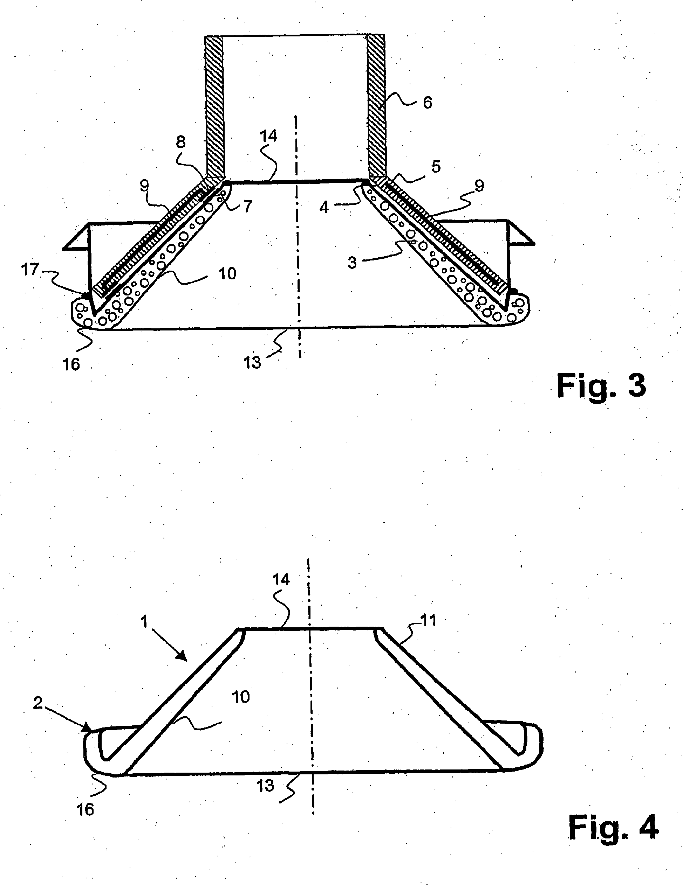

In Figur 3 ist ein weiteres Ausführungsbeispiel der Erfindung dargestellt. Hier weist der Brusthaubeneinsatz Auslösemittel 7 auf, welche im in die Haube eingelegten Zustand Schaltermittel 8 der Brusthaube 6 kontaktieren bzw. aktivieren. Dadurch wird eine in der Brusthaube 6 angeordnete Heizung 9 betätigt. Die Heizung 9 besteht vorzugsweise aus Widerstandsheizdrähten. Die Heizung 9 gibt über Wärmestrahlung und -leitung Wärme an den Brusthaubeneinsatz ab, so dass sich dieser wiederum erwärmt bzw. die Wärme an die Brust weiterleitet. Als Auslösemittel 7 und Schaltermittel 8 eignen sich beispielsweise elektrische Kontaktflächen, insbesondere Metallplättchen, welche einen Stromkreis schliessen. Es lassen sich jedoch auch Stift/Steckerverbindungen oder andere geeignete Mittel verwenden, um einen Stromkreis zu schliessen und die Heizung 9 zu aktivieren. Es ist auch möglich, dass die Haube oder ein anderes Teil der Brustpumpe mit einem Schalter versehen ist, um eine Heizung in der Haube zu aktivieren, ohne dass der Brusthaubeneinsatz Auslösemittel benötigt.

Auch bei der Ausführungsform gemäss Figur 3 ist je nach Art der Verwendung ein hoher Wärmeleitungskoeffizient und/oder eine hohe Wärmespeicherkapazität des Grundkörpers 1 notwendig.

Die Ausführungsformen mit einer in der Haube integrierten Heizung weisen den Vorteil auf, dass der Brusthaubeneinsatz, welcher ein Einwegprodukt ist, relativ kostengünstig gefertigt werden kann.

Der Brusthaubeneinsatz ist in den oben beschriebenen Figuren lediglich schematisch dargestellt, insbesondere das Zusammenwirken der Brusthaube mit dem Brusthaubeneinsatz ist nur schematisch gezeichnet. In Wirklichkeit ist der Kragen 2 um das körperabgewandte vordere Ende der Brusthaube 6 gestülpt und liegt in diesem vorderen Bereich der Brusthaube auch auf der äusseren Oberfläche 60 des Trichters der Brusthaube 6 auf. Dadurch ist es auch möglich, die Kontaktelemente 5 bzw. die Schaltermittel 8 auf dieser äusseren Seite der Brusthaube anzuordnen und die Kontaktmittel 4 bzw. die Auslösemittel 7 an der Innenseite des Kragens 2 anzuordnen. Diese Anordnung hat den Vorteil, dass diese Elemente und Mittel nicht zwischen Haube und Brust zu liegen kommen und deshalb nicht als Druckstellen für die empfindliche Brust wirken können.

Die oben beschriebenen Ausführungsformen sind vorzugsweise doppelwandig ausgeführt und weisen die mit einem Füllmaterial gefüllte Kammer 12 auf. Sie bzw. ihre Grundkörper können jedoch auch einstückig aus einem geeigneten weichen und verformbaren Material geformt sein. Insbesondere die Ausführungsformen mit im Brusthaubeneinsatz oder in der Brusthaube integrierter Heizung benötigen nicht zwingend eine gelgefüllte Kammer.

In Figur 4 ist ein weiteres Ausführungsbeispiel eines erfindungsgemässen Brusthaubeneinsatzes dargestellt. Der Einsatz weist im wesentlichen dieselbe Form auf wie oben beschrieben. Auch er weist einen Kragen 2 auf, welcher über ein vorderes Ende einer Brusthaube gestülpt werden kann, um den Einsatz im Trichter der Brusthaube zu halten. Er ist jedoch einstückig aus einem relativ weichen, aber doch formstabilen Schaumstoff oder einem entsprechenden schaumstoffähnlichen Material, insbesondere aus Polyurethan-Schaum oder einem Polyolefine-Schaum gefertigt.

Der erfindungsgemässe Brusthaubeneinsatz sowie die erfindungsgemässe Brusthaube für eine Brustpumpe erhöhen den Komfort beim Gebrauch und optimieren den Abpumpvorgang.

### Bezugszeichenliste

- 1: Grundkörper
- 10: innere Kegelwand
- 11: äussere Kegelwand
- 22: Kammer
- 13: Grosse Öffnung
- 14: Kleine Öffnung
- 15: Füllmedium
- 16: Auflagepolster
- 17: Schweissnaht

- 2: Kragen
- 20: erster Schenkel
- 21: zweiter Schenkel

- 3: Widerstandsheizdraht

- 4: Kontaktmittel

- 5: Kontaktelemente

- 6: Brusthaube

- 7: Auslösemittel

- 8: Schaltermittel

- 9: Heizung

## Patentansprüche

1. Brusthaubeneinsatz für eine Brustpumpe, welcher im Gebrauch zum Anliegen an eine Areola einer weiblichen Brust vorgesehen ist, wobei der Brusthaubeneinsatz einen kegelstumpfförmigen Grundkörper (1) mit einer grossen hinteren Öffnung (13) und einer kleinen vorderen Öffnung (14) aufweist, welcher zur Aufnahme in einen Trichter einer Brusthaube (6) ausgebildet ist, wobei der kegelstumpfförmige Grundkörper (1) mindestens teilweise aus einem wärmespeichernden und/oder wärmeleitenden Material gefertigt ist und wobei er im Gebrauch erwärmt ist, wobei diese Erwärmung nicht ausschliesslich durch Übermittlung von Körpertemperatur der weiblichen Brust erfolgt, **dadurch gekennzeichnet, dass** der Grundkörper (1) mit mindestens einem Widerstandsheizelement (3) versehen ist und der Einsatz mit dem mindestens einen Widerstandsheizelement (3) verbundene elektrische Kontaktmittel (4) aufweist.

2. Brusthaubeneinsatz für eine Brustpumpe, welcher im Gebrauch zum Anliegen an eine Areola einer weiblichen Brust vorgesehen ist, wobei der Brusthaubeneinsatz einen kegelstumpfförmigen Grundkörper (1) mit einer grossen hinteren Öffnung (13) und einer kleinen vorderen Öffnung (14) aufweist, welcher zur Aufnahme in einen Trichter einer Brusthaube (6) ausgebildet ist, wobei der kegelstumpfförmige Grundkörper (1) mindestens teilweise aus einem wärmespeichernden und/oder wärmeleitenden Material gefertigt ist und wobei er im Gebrauch erwärmt ist, wobei diese Erwärmung nicht ausschliesslich durch Übermittlung von Körpertemperatur der weiblichen Brust erfolgt, **dadurch gekennzeichnet, dass** der Brusthaubeneinsatz mit Auslösemitteln (7) versehen ist, um bei Gebrauch eine im Trichter der Brusthaube (6) angeordnete Heizung (9) zu aktivieren.

3. Brusthaubeneinsatz nach einem der Ansprüche 1 oder 2, wobei der Grundkörper (1) eine innere und eine äussere Kegelwand (10, 11) aufweist, welche eine Kammer (12) bilden, wobei die Kammer (12) mit einem wärmespeichernden und/oder wärmeleitenden Füllmedium (15) versehen ist.

4. Brusthaubeneinsatz nach Anspruch 3, wobei mindestens die innere Kegelwand (10) elastisch deformierbar ausgebildet ist.

5. Brusthaubeneinsatz nach einem der Ansprüche 3 oder 4, wobei das Füllmedium (15) ein Gel ist.

6. Brusthaubeneinsatz nach einem der Ansprüche 1 bis 5, wobei er ein Mittel (2) zum Befestigen des Grundkörpers (1) im Trichter der Brusthaube (2) aufweist.

7. Brusthaubeneinsatz nach Anspruch 6, wobei das Mittel zum Befestigen des Grundkörpers (1) ein die grosse Öffnung (13) umlaufender Kragen (2) ist.

8. Brusthaubenset einer Brustpumpe mit einer Brusthaube und einem Brusthaubeineinsatz gemäss Anspruch 1, wobei die Brusthaube elektrische Kontaktmittel (5) zur Kontaktierung der Kontaktmittel (4) des Einsatzes aufweist.

9. Brusthaubenset einer Brustpumpe mit einer Brusthaube und einem Brusthaubeineinsatz gemäss Anspruch 2, wobei die Brusthaube eine Heizung (9) zur Erwärmung des Einsatzes im in die Brusthaube eingelegten Zustand und mit der Heizung (9) verbundene Schaltermittel (8) zur Kontaktierung des Auslösemittels (7) des Einsatzes aufweist.

## Claims

1. A breastshield insert for a breast pump, which breastshield insert is designed to lie on an areola of a female breast during use, said breastshield insert comprising a frustoconical base body (1) with a large rear opening (13) and a small front opening (14), said base body (1) being designed to be received in a funnel of a breastshield (6), wherein the frustoconical base body (1) is at least partly made of a heat-accumulating and/or heat-conducting material and wherein it is warmed during use, this warming not exclusively taking place through transfer of body temperature from the female breast, **characterized in that** the base body (1) is provided with at least one resistance heating element (3), and the insert has electrical contact means (4) connected to the at least one resistance heating element (3).

2. A breastshield insert for a breast pump, which breastshield insert is designed to lie on an areola of a female breast during use, said breastshield insert comprising a frustoconical base body (1) with a large rear opening (13) and a small front opening (14), said base body (1) being designed to be received in a funnel of a breastshield (6), wherein the frustoconical base body (1) is at least partly made of a heat-accumulating and/or heat-conducting material and wherein it is warmed during use, this warming not exclusively taking place through transfer of body temperature from the female breast, **characterized in that** the breastshield insert is provided with trigger means (7) for activating a heating system (9) arranged in the funnel of the breastshield (6) during use.

3. The breastshield insert as claimed in claim 1 or 2, in which the base body (1) has an inner cone wall (10) and outer cone wall (11) which form a chamber (12), the chamber (12) being provided with a heat-accumulating and/or heat-conducting filler medium (15).

4. The breastshield insert as claimed in claim 3, in which at least the inner cone wall (10) is elastically deformable.

5. The breastshield insert as claimed in either of claims 3 or 4, in which the filler medium (15) is a gel.

6. The breastshield insert as claimed in one of claims 1 through 5, with means (2) for securing the base body (1) in the funnel of the breastshield (6).

7. The breastshield insert as claimed in claim 6, in which the means for securing the base body (1) is a collar (2) surrounding the large opening (13).

8. A set of a breast pump with a breastshield and a breastshield insert as claimed in claim 1, in which the breastshield has electrical contact means (5) for making contact with contact means (4) of the insert.

9. A set of a breast pump with a breastshield and a breastshield insert as claimed in claim 2, in which the breastshield has a heating system (9) for warming the insert fitted in the breastshield, and has switch means (8) which are connected to the heating system (9) and make contact with the trigger means (7) of the insert.

## Revendications

1. Embout pour téterelle pour un tire-lait, qui est prévu, pendant l'utilisation, pour s'appliquer contre l'aréole d'un sein d'une femme, l'embout pour téterelle présentant un corps de base de forme tronconique (1) avec une grande ouverture arrière (13) et une petite ouverture avant (14), qui est réalisé pour recevoir dans un entonnoir une téterelle (6), le corps de base de forme tronconique (1) étant au moins en partie fabriqué en un matériau accumulateur de chaleur et/ou thermoconducteur et étant chauffé pendant l'utilisation, ce chauffage ne s'effectuant pas exclusivement par transmission de la température corporelle du sein de la femme, **caractérisé en ce que** le corps de base (1) est muni d'au moins un élément de chauffage à résistance (3) et l'embout présente des moyens de contact électrique (4) connectés à l'au moins un élément de chauffage à résistance (3).

2. Embout pour téterelle pour un tire-lait, qui est prévu, pendant l'utilisation, pour s'appliquer contre l'aréole d'un sein d'une femme, l'embout pour téterelle présentant un corps de base de forme tronconique (1) avec une grande ouverture arrière (13) et une petite ouverture avant (14), qui est réalisé pour recevoir dans un entonnoir une téterelle (6), le corps de base de forme tronconique (1) étant au moins en partie fabriqué en un matériau accumulateur de chaleur et/ou thermoconducteur et étant chauffé pendant l'utilisation, ce chauffage ne s'effectuant pas exclusivement par transmission de la température corporelle du sein de la femme, **caractérisé en ce que** l'embout pour téterelle est muni de moyens de déclenchement (7) pour, pendant l'utilisation, activer un chauffage (9) disposé dans l'entonnoir de la téterelle (6).

3. Embout pour téterelle selon la revendication 1 ou 2, dans lequel le corps de base (1) présente une paroi conique interne et une paroi conique externe (10, 11) qui forment une chambre (12), la chambre (12) étant munie d'un milieu de remplissage (15) accumulateur de chaleur et/ou thermoconducteur.

4. Embout pour téterelle selon la revendication 3, dans lequel au moins la paroi conique interne (10) est réalisée de manière déformable élastiquement.

5. Embout pour téterelle selon l'une quelconque des revendications 3 ou 4, dans lequel le milieu de remplissage (15) est un gel.

6. Embout pour téterelle selon l'une quelconque des revendications 1 à 5, celui-ci présentant un moyen (2) pour fixer le corps de base (1) dans l'entonnoir de la téterelle (2).

7. Embout pour téterelle selon la revendication 6, dans lequel le moyen pour fixer le corps de base (1) est un collet (2) s'étendant autour de la grande ouverture (13).

8. Ensemble de téterelle d'un tire-lait comprenant une téterelle et un embout pour téterelle selon la revendication 1, la téterelle présentant des moyens de contact électrique (5) pour le contact avec les moyens de contact (4) de l'embout.

9. Ensemble de téterelle d'un tire-lait comprenant une téterelle et un embout pour téterelle selon la revendication 2, dans lequel la téterelle présente un chauffage (9) pour chauffer l'embout dans l'état installé dans la téterelle et des moyens de commutation (8) connectés au chauffage (9) pour le contact du moyen de déclenchement (7) de l'embout.
